# EUROPEAN PATENT APPLICATION

(11) **EP 1 415 594 A1**
(43) Date of publication of application: **06.05.2004**
(21) Application number: 03024607.8
(22) Date of filing: 25.10.2003
(51) Int. Cl.: A61B 6/00

(54) **System, apparatus and method of radiographing medical image and program**

(30) Priority: 31.10.2002 JP 2002317228
(71) Applicant: KONICA MINOLTA HOLDINGS, INC., Tokyo 100-0005 (JP)
(72) Inventor: Moriyama, Naoto, Hachioji-shi Tokyo 192-8505 (JP)
(74) Representative: Gille Hrabal Struck Neidlein Prop Roos

(57) **Abstract**

A medical image radiographing system can improve the safety and reliability in the medical image radiographing by managing medical treatment acts of an operator while improving the security of a medical image radiographing apparatus. The medical image radiographing apparatus of the medical image radiographing system has an irradiation section to irradiate a subject with the radiations, an obtaining section to obtain identification information of a radiographer and a radiographing instruction, and a radiographing operation control section to control the irradiation section to perform a radiographing operation according to the radiographing instruction obtained by the obtaining section when the radiographer is authenticated according to the identification information of the radiographer obtained by the obtaining section.

## Description

### BACKGROUND OF THE INVENTION

### Field of the Invention

The present invention relates to a system, an apparatus and a method of radiographing a medical image and a program.

### Description of Related Art

In a field of medicine, a medical image radiographing (or tomographing) system having a computed tomographing apparatus (hereinafter, named "computed tomography (CT)"), a computed radiation image radiographing apparatus (hereinafter, named "computed radiography (CR)"), a nuclear magnetic resonance imaging apparatus (hereinafter, named "magnetic resonance imaging (MRI)") or the like has been used. In this medical image radiographing system, a patient denoting a subject is radiographed, and a radiographed medical image is obtained as digital image data.

Particularly, in the CR, a medical image conversion panel with a photostimulable phosphor layer formed on a support member is in use. The photostimulable phosphor layer of the medical image conversion panel absorbs radiations transmitted through the subject to accumulate the energy of the radiations corresponding to the radiation transmittance of each part of the subject in the photostimulable phosphor layer and to form a latent image in the photostimulable phosphor layer. Thereafter, the photostimulable phosphor layer is scanned by excitation light such as infrared rays or the like to release the accumulated energy of the radiations as fluorescence, and the fluorescence is photoelectrically converted to obtain a signal of a medical image. Image processing is performed for the obtained medical image. Thereafter, the processed medical image is output to a film, a cathode ray tube (CRT) or the like to be visualized, or is stored in a filing device such as a server or the like along with patient information. Thereby, the output medical image or the stored medical image can be used for medical service.

The medical image radiographing system using the CR is classified into two system types. One system type is an earlier developed medical image radiographing system in which both an apparatus for radiographing a subject to read out an image of the subject and a photostimulable phosphor plate are fixedly arranged in a radiographing room, and the radiographing is performed in the radiographing room. In this system, the radiographing of the subject and the reading-out of the image can be performed together in the radiographing room.

The other system type is a medical image radiographing system recently widely used for patients such as patients having broken bones, patients having diseases of cerebral blood vessels, patients placed in intensive care units and/or the like. In this system, because the radiographing cannot be performed for the patients in the radiographing room, a movable radiographing apparatus applied to a doctor's round of visits and a portable cassette containing a phosphor plate are used, and the radiographing of each patient is performed at a visited patient's position. In this system, after the radiographing, the cassette is inserted into a reader only used for the cassette, and the obtained medical image is read out.

The procedure of the radiographing performed by an operator in the medical image radiographing system relating to the doctor's round of visits will be described. The operator initially prints out radiographing order information prepared according to a doctor's instruction on an instruction sheet by using an information management system of a hospital (hereinafter, named "hospital information system (HIS)") or an information management system of a radiation treatment department (hereinafter, named "radiology information system (RIS)"). The radiographing order information includes information (hereinafter, named "patient information") relating to each radiographed patient such as a name, a sex and the like and information (hereinafter, named "radiographing information") relating to the radiographing such as a radiographic part of the patient, a radiographing method and the like. Therefore, the radiographing order information has the instruction indicating which type of radiographing is to be performed for each patient.

A movable radiographing apparatus, a cassette and the instruction sheet are then transferred to a hospital room in which a patient to be radiographed stays, and the patient is identified according to the radiographing order information printed out on the instruction sheet. After successfully identifying the patient, the operator operates the radiographing apparatus to radiograph the patient, and records a medical image of the patient in the cassette. After completing the radiographing, the operator inserts the cassette having the recorded medical image into the reader to read out the medical image from the cassette.

As described above, when the instruction indicated by the doctor is correctly transferred to each patient's position and the radiographing is carefully performed according to the instruction, the medical image of each patient can be correctly obtained. However, if wrong medical treatment is performed by radiographing a wrong patient or radiographing a patient according to a radiographing instruction to radiograph another patient, it is difficult to specify the cause of the occurrence of the wrong medical treatment in a medical treatment place in which many medical treatment stuffs such as a doctor(s), a nurse(s), an operator(s) (or a radiographer(s)), a therapist(s), a pharmacist(s) and the like work together. Therefore, to prevent the wrong medical treatment, there is provided a well-known medical application portable terminal (for example, refer to a patent document disclosed in Unexamined Japanese Patent Publication (Tokkai) 2002-83065) to record medical treatment acts performed by a medical performer(s) such as a doctor(s), a nurse(s) and/or the like. Accordingly, each medical treatment act performed by the medical performers for each patient can be made clear, and the occurrence of the wrong medical treatment can be suppressed.

However, in the earlier developed medical image radiographing systems, medical treatment acts of the operator are hardly managed by the medical application portable terminal. Further, the movable medical image radiographing apparatus described above is usually used in the outside of the radiographing room, and persons other than the operators can operate the radiographing apparatus. Therefore, there is a probability that a third person erroneously operates the radiographing apparatus. Because the medical image radiographing apparatus irradiates a human body with X-rays exerting adverse influence on the human body, the radiographing apparatus requires to be handled safely and accurately.

### SUMMARY OF THE INVENTION

In instruction to solve the above problem, an object of the present invention is to provide a medical image radiographing system, a medical image radiographing apparatus, a medical image radiographing method and a program, which manage a medical treatment act of an operator at high safety and reliability while improving the security of the medical image radiographing apparatus.

In accordance with the first aspect of the present invention, a medical image radiographing system comprises:
a medical image radiographing apparatus to perform radiographing of a medical image by irradiating with radiations,
the medical image radiographing apparatus comprising:
an irradiation section to irradiate a subject with the radiations;
an obtaining section to obtain identification information of a radiographer and a radiographing instruction; and
a radiographing operation control section to control the irradiation section to perform a radiographing operation according to the radiographing instruction obtained by the obtaining section when the radiographer is authenticated according to the identification information of the radiographer obtained by the obtaining section.

In accordance with the second aspect of the present invention, a medical image radiographing apparatus to perform the radiographing of a medical image by irradiating with radiations, the medical image radiographing apparatus comprises:
an irradiation section to irradiate a subject with the radiations;
an obtaining section to obtain identification information of a radiographer and a radiographing instruction; and
a radiographing operation control section to control the irradiation section to perform a radiographing operation according to the radiographing instruction obtained by the obtaining section when the radiographer is authenticated according to the identification information of the radiographer obtained by the obtaining section.

In accordance with the third aspect of the present invention, a medical image radiographing method for radiographing a medical image by irradiating with radiations, the method comprises:
obtaining identification information of a radiographer;
performing authentication of the radiographer according to the obtained identification information of the radiographer;
obtaining a radiographing instruction; and
performing a radiographing operation according to the obtained radiographing instruction by irradiating with the radiations when the radiographer is authenticated.

In accordance with the fourth aspect of the present invention, a program makes a computer
obtain identification information of a radiographer and a radiographing instruction;
perform authentication of the radiographer according to the obtained identification information of the radiographer; and
instruct to perform a radiographing operation according to the obtained radiographing instruction by irradiating with radiations when the radiographer is authenticated.

In the system in accordance with the first aspect of the present invention, the apparatus in accordance with the second aspect of the present invention, the method in accordance with the third aspect of the present invention and the program in accordance with the fourth aspect of the present invention, when the medical image radiographing apparatus is operated, the medical image radiographing apparatus obtains the identification information of the radiographer. When the medical image radiographing apparatus succeeds in the authentication of the radiographer, the radiographing of the medical image is performed according to the radiographing instruction. Accordingly, the radiographing of the medical image according to a radiographing instruction other than the radiographing instruction registered as the radiographer in advance is prohibited, and the medical image radiographing apparatus can be prevented from being carelessly operated by a third person.

Preferably, in the medical image radiographing apparatus of the system, the obtaining section obtains the identification information of the radiographer and the radiographing instruction by inputting the identification information and the radiographing instruction.

Preferably, the system further comprises a medical image terminal,
the medical image terminal comprising:
an input section to input the identificafion information of the radiographer; and
a transmission section to transmit the identification information of the radiographer inputted by the input section,
wherein the obtaining section obtains the identification information of the radiographer by receiving the identification information transmitted by the transmission section and obtains the radiographing instruction by inputting the radiographing instruction.

Preferably, the system further comprises a medical image terminal,
the medical image terminal comprising:
an input section to input the identification information of the radiographer and the radiographing instruction; and
a transmission section to transmit the identification information of the radiographer and the radiographing instruction inputted by the input section,
wherein the obtaining section obtains the identification information of the radiographer and the radiographing instruction by receiving the identification information and the radiographing instruction transmitted by the transmission section.

Preferably, in the apparatus, the obtaining section obtains the identification information of the radiographer and the radiographing instruction by inputting the identification information and the radiographing instruction.

Preferably, in the apparatus, the obtaining section obtains the identification information of the radiographer by receiving the identification information and obtains the radiographing instruction by inputting the radiographing instruction.

Preferably, in the apparatus, the obtaining section obtains the identification information of the radiographer and the radiographing instruction by receiving the identification information and the radiographing instruction.

Therefore, when the radiographer is not authenticated, even though the radiographing instruction is inputted from the input section of the medical image radiographing apparatus or the medical image terminal, the radiographing operation cannot be performed. Accordingly, the medical image radiographing apparatus can be prevented from being erroneously operated by a third person, and the medical image radiographing apparatus can be safely handled.

Preferably, in the medical image terminal of the system, the input section of the medical image terminal inputs a radiographing condition, the transmitting section of the medical image terminal transmits the radiographing condition, the obtaining section of the medical image radiographing apparatus obtains the radiographing condition by receiving the radiographing condition from the transmitting section, and the radiographing operation control section of the medical image radiographing apparatus controls the irradiation section to perform the radiographing operation according to the radiographing condition obtained by the obtaining section and the obtained radiographing instruction.

Preferably, in the apparatus, the obtaining section obtains a radiographing condition by inputting the radiographing condition, and the radiographing operation control section controls the irradiation section to perform the radiographing operation according to the radiographing condition and the radiographing instruction obtained by the obtaining section.

Preferably, the method further comprises:
obtaining a radiographing condition, and
the performing of the radiographing operation includes performing the radiographing operation according to the obtained radiographing instruction and the obtained radiographing condition by irradiating with the radiations when the radiographer is authenticated.

Preferably, the method further comprises:
obtaining a radiographing condition, and
the performing of the radiographing operation includes performing the radiographing operation according to the obtained radiographing instruction and the obtained radiographing condition by irradiating with the radiations when the radiographer is authenticated.

Preferably, the program further makes the computer obtain a radiographing condition and instruct to perform the radiographing operation according to the radiographing condition and the radiographing instruction by irradiating with the radiations.

In the system, the apparatus, the method and the program, because the radiographing condition can be inputted to the medical image radiographing apparatus through the medical image terminal, the operability of the medical image radiographing apparatus is superior. Moreover, because the input section of the medical image radiographing apparatus can be miniaturized, when the radiographing is performed by carrying the medical image radiographing apparatus to the visited patients position, the medical image radiographing apparatus can be easily carried, and the radiographing of the medical image can be quickly performed.

Preferably, the medical image radiographing apparatus further comprises a storage to store radiographing history information including the radiographing condition of the radiographing operation while setting the correspondence of the radiographing history information to the identification information of the radiographer when the radiographing operation is performed under control of the radiographing operation control section.

Preferably, the apparatus further comprises a storage to store radiographing history information including a radiographing condition of the radiographing operation while setting the correspondence of the radiographing history information to the identification information of the radiographer when the radiographing operation is performed under control of the radiographing operation control section.

Preferably, the method further comprises the storing of radiographing history information including the radiographing condition of the radiographing operation while setting the correspondence of the radiographing history information to the identification information of the radiographer when the radiographing operation is performed.

Preferably, the program further makes the computer perform the storing of radiographing history information including the radiographing condition of the radiographing operation while setting the correspondence of the radiographing history information to the identification information of the radiographer when the radiographing operation is performed.

In the system, the apparatus, the method and the program, because the history of the radiographing performed by the radiographer is stored while setting the correspondence to the identification information of the radiographer, when wrong medical treatment is performed, the cause of the occurrence of the wrong medical treatment can be immediately examined, and the recurrence of the wrong medical treatment can be prevented. Moreover, because the history of the radiographing performed by the radiographer is stored, the effect of the suppression of the occurrence of the wrong medical treatment can be expected to the radiographer.

Preferably, in the system, the medical image terminal is a portable terminal.

In the system, because the medical image terminal is a portable terminal, each of the radiographers can bring his/her own medical image terminal. Accordingly, only the specific radiographer can operate his/her medical image terminal, and the security of the medical image radiographing system can be improved. Moreover, when the radiographing is performed at the visited patient's position, radiographing members can be easily carried by miniaturizing the medical image terminal, and the burden of the radiographer can be mitigated.

It will be appreciated that each of fhe irradiation section, the obtaining section, the radiographing operation control section and the like in the medical image radiographing apparatus may comprise a single unit or a plurality of units. Further, it will be appreciated that one unit or units are arranged in common in two sections or more selected from the irradiation section, the obtaining section, the radiographing operation control section and the like. As an example that one unit or units are arranged in common in two sections or more, the obtaining section comprises a keyboard and a central processing unit (CPU), and the CPU is used as a unit of the radiographing operation control section.

It will be appreciated that each of the input section, the transmission section and the like in the medical image terminal may comprise a single unit or a plurality of units. Further, it will be appreciated that one unit or units are arranged in common in two sections or more selected from the input section, the transmission section and the like. As an example that one unit or units are arranged in common in two sections or more, the input section comprises a barcode reader with transmission function and a communication unit, and the communication unit is used as a unit of the transmission section.

### BRIEF DESCRIPTION OF THE DRAWINGS

The present invention will become more fully understood from the detailed description given hereinbelow and the accompanying drawing which are given by way of illustration only, and thus are not intended as a definition of the limits of the present invention, and wherein;
FIG. 1 is a conceptual view showing the configuration of a medical image radiographing system according to the embodiment of the present invention;
FIG. 2 is a block diagram showing the functional configuration of a portable radiographing information apparatus shown in FIG. 1;
FIG. 3 is a block diagram showing the functional configuration of a radiographing apparatus shown in FIG. 1;
FIG. 4 is a view showing an example of the data construction of an operator management file stored in a storage of FIG. 3;
FIG. 5 is a flow chart showing the medical image radiographing processing performed by a CPU of FIG. 2; and
FIG. 6 is a flow chart showing the medical image radiographing processing performed by a CPU of FIG. 3.

### PREFERRED EMBODIMENTS OF THE INVENTION

An embodiment of the present invention will be explained hereinafter with reference to the drawings. A medical image terminal according to the present invention is, as an example, applied to a portable radiographing information apparatus (hereinafter, named "Personal Digital Assistant (PDA)").

The configuration of a medical image radiographing system is initially described.

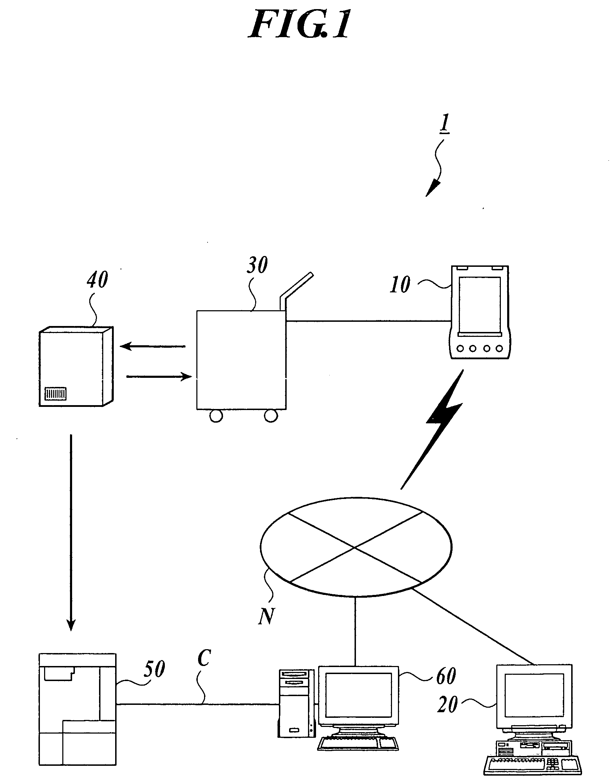

FIG. 1 is a conceptual view showing the configuration of a medical image radiographing system 1 according to the embodiment of the present invention. As shown in FIG. 1, the medical image radiographing system 1 comprises a PDA 10, an information management apparatus 20, a medical image radiographing apparatus 30, a cassette 40, a medical image reader 50 and a control section 60. The PDA 10, the information management apparatus 20 and the control section 60 are connected with one another through a network N so as to be able to gain access to one another.

The PDA 10 is a portable radiographing information apparatus which is carried by a radiographing operator operating the medical image radiographing apparatus 30. The PDA 10 is connected to the medical image radiographing apparatus 30 through a cable B to authenticate an operator (or radiographer) in cooperation with the medical image radiographing apparatus 30 and to transmit radiographing conditions and a radiographing instruction to the medical image radiographing apparatus 30. Further the PDA 10 receives radiographing order information from the information management apparatus 20 through the network N.

The information management apparatus 20 acts as a terminal to manage pieces of radiographing order information based on the radiographing instructions of a doctor(s), extracts radiographing order information corresponding to a radiographing instruction transmitted from the PDA 10 and transmits the extracted radiographing order information to the PDA 10 through the network N. A reception apparatus (not shown) to receive the reservation of the radiographing order information may be applied as another information management apparatus, and an information management system such as a hospital information system (HIS), a radiology information system (RIS) or the like may be applied as another information management apparatus.

The medical image radiographing apparatus (hereinafter, abbreviated to "radiographing apparatus") 30 is a movable radiographing apparatus, radiographs each of patients at a visited patient's position and records a medical image of the patient in the cassette 40 detachable from the radiographing apparatus 30. The cassette 40 has a photostimulable phosphor sheet in which energy of a portion of radiations can be accumulated. The radiations are output from an irradiation source and are transmitted through a subject placed between the irradiation source and the cassette 40, and energy of a portion of the radiations is accumulated in the photostimulable phosphor sheet.

The medical image reader 50 reads out the medical image recorded in the cassette 40. In detail, the medical image reader 50 irradiates the photostimulable phosphor sheet of the cassette 40 with excitation light, photoelectrically converts stimulated light emitted from the sheet and performs analog-to-digital conversion for an obtained image signal to obtain the medical image.

The control section 60 controls a read-out operation of the medical image reader 50. In detail, the control section 60 sets the correspondence of the medical image read out from the cassette 40 to the radiographing order information received from the information management apparatus 20 and stores the medical image and the radiographing order information in a data base to manage the medical image.

Various networks such as a local area network (LAN), a wide area network (WAN), an internet and the like can be applied to the network N. Further, wireless communication or infrared communication may be applied to the network N on condition that the communication is allowed in medical institutions such as a hospital and the like. However, because the radiographing order information includes important patient information, the radiographing order information is preferably enciphered in the transmission and reception of the radiographing order information.

Next, main constitutional elements of the medical image radiographing system 1 according to the present invention will be described in detail.

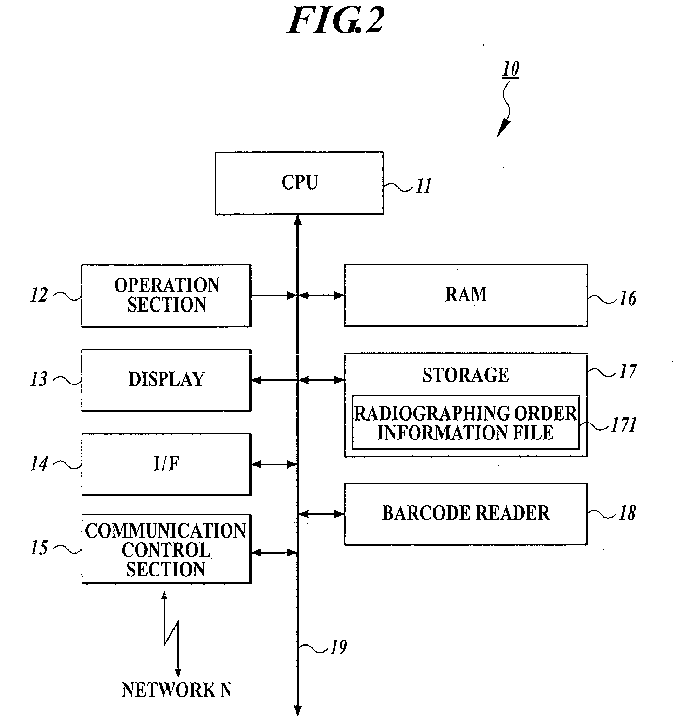

FIG. 2 is a block diagram showing the functional configuration of the PDA 10. As shown in FIG. 2, the PDA 10 comprises a central processing unit (CPU) 11, an operation section 12, a display 13, a communication control section 15, a random access memory (RAM) 16, a storage 17 and a barcode reader 18. Each of the constituent elements of the PDA 10 is connected to a bus 19.

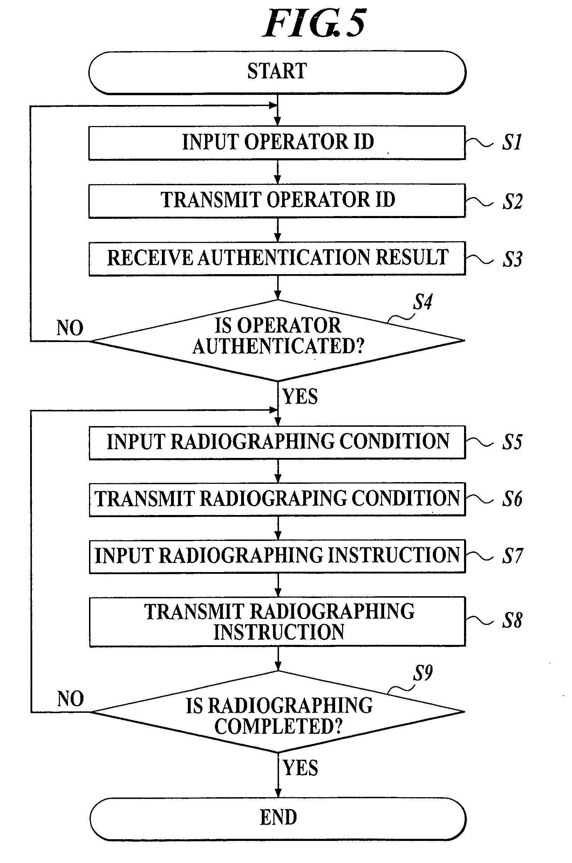

The CPU 11 expands a program which is designated and selected from a system program and various types of application programs stored in the storage 17 and controls intensively each element of the PDA 10 according to the designated program. In detail, the CPU 11 loads a medical image radiographing processing program from the storage 17 and performs medical image radiographing processing (refer to FIG. 5) described later.

To perform the medical image radiographing processing, when the CPU 11 receives an operator identification number (ID) through the operation section 12 or the barcode reader 18, the CPU 11 transmits the operator ID to the radiographing apparatus 30 connected to the cable B through an interface (I/F) 14. Further, the CPU 11 receives an authentication result from the radiographing apparatus 30 through the I/F 14 and judges according to the authentication result whether or not the radiographing performed by an operator is allowed. When the operator fails in authentication, the CPU 11 displays an error screen on the display 13 to urge the operator to again input his operator ID. When the operator fails in authentication a predetermined number of times (for example, three times), the CPU 11 finishes the medical image radiographing processing.

When the operator is successfully authenticated, the CPU 11 urges the operator to input a radiographing condition(s) through the operation section 12. The radiographing conditions include detailed conditions such as information of resolution, sensitivity, a radiographing size, a radiographic part, a radiographing direction, a radiation dose of X-rays and the like, in addition to radiographing information included in the radiographing order information. In the transmission of the radiographing conditions, the radiographing order information obtained from the storage 17 may be transmitted with the radiographing conditions. When the CPU 11 receives a radiographing instruction from the operator through the operation section 12, the CPU 11 transmits the received radiographing instruction to the radiographing apparatus 30 to make the radiographing apparatus 30 radiograph a subject.

The operation section 12 is an example of an input section described according to the present invention and comprises a cursor key, numeral input keys and various functional keys. The operation section 12 outputs an operation signal corresponding to a key operated by the operator. The operation section 12 receives, for example, the operator ID denoting identification information of the operator in response to the manipulation of the operator. The operation section 12 may further comprises a pointing device such as a touch panel or the like or another input apparatus, if necessary.

The display 13 comprises a liquid crystal display (LCD) or the like and displays various types of information such as the radiographing order information, the operator ID and the like according to a display instruction transmitted from the CPU 11.

The I/F 14 is an example of a transmission section described according to the present invention and adjusts the form and the transfer rate of data transmitted and received through the cable B to intermediate the transmission and reception of the data between the radiographing apparatus 30 and the PDA 10. The connection of the I/F 14 with the radiographing apparatus 30 is not limited to a wire, and the I/F 14 may be connected with the radiographing apparatus 30 in wireless communication. In this case, the I/F 14 has a data communication control function to control a data communication protocol according to a data communication method such as the Infrared Data Association (IrDA) or the like and performs short-range communication by using infrared rays. The communication method performing the wireless communication is not limited to IrDA, and a Bluetooth method may be adopted as the communication method performing the wireless communication.

A reception section and a transmission section of the communication control section 15 comprise a network interface card, a modem or a terminal adapter and the like, and the communication control section 15 controls the communication with an external apparatus through the network N. For example, the communication control section 15 establishes the wireless communication with the information management apparatus 20, transmits an instruction indicating a request of the radiographing order information through the network N and receives the radiographing order information from the information management apparatus 20. In this case, if necessary, the wireless communication may be established by using a portable telephone terminal such as Personal Handyphone System (PHS) or the like.

The RAM 16 has a work memory area to store the designated application program described before, an inputted instruction, input data, processing results and the like.

The storage 17 comprises a storage medium (not shown) in which programs and data are stored in advance. The storage medium stores a system program, various application programs corresponding to the system program and data obtained by executing each of various processing programs. The storage medium comprises a magnetic or optical storage medium or a semiconductor memory and is attached to the storage 17 fixedly or detachably.

The storage 17 stores a radiographing order information file 171 to store pieces of radiographing order information received from the information management apparatus 20. The radiographing order information file 171 has columns of a radiographing instruction identification number (ID), a patient ID, a name, a sex, an age, a hospital room, a department in charge, a radiographic part, a radiographing direction, the number of radiographing sheets and a cassette ID and stores data of the columns for each piece of radiographing order information.

The radiographing order information has the patient information and the radiographing information. The patient information of each patient may comprise, for example, a name of a doctor in charge of the patient, warning information indicating the warning of infectious diseases and the like, the/no existence of a drug allergy, pregnancy or not, a clinical history, the/no necessity of special nursing such as the/no necessity of a wheel chair, the/no necessity of a stretcher or the like, a clinically diagnosed disease, privileged information to be protected and the like, in addition to a patient ID, a name, a sex and an age of the patient. The radiographing information may comprises, for example, a radiographing method (simple radiography, contrast radiography or the like), an expected date of the radiographing and the like, in addition to information of a radiographic part, a radiographing direction and a radiographing apparatus and the number of radiographing sheets.

The barcode reader 18 is an example of an input section to receive the operator ID denoting identification information of the operator and has a scanner denoting an optical reader. The scanner reads out a barcode, and information indicated by the barcode is obtained by decoding the barcode according to a predetermined standard. In detail, the barcode reader 18 reads out a barcode attached to an ID card of the operator to obtain the operator ID, reads out a barcode attached to a bed side of the patient or a part of the patient body to obtain the patient ID, and reads out a barcode attached to the cassette recording the medical image to obtain the cassette ID. The predetermined standard denotes JAN code, UPC code, CODE 39, CDE 93, CODE 128, NW-7, INDUSTRIAL 2 of 5, ITF physical distribution code or the like.

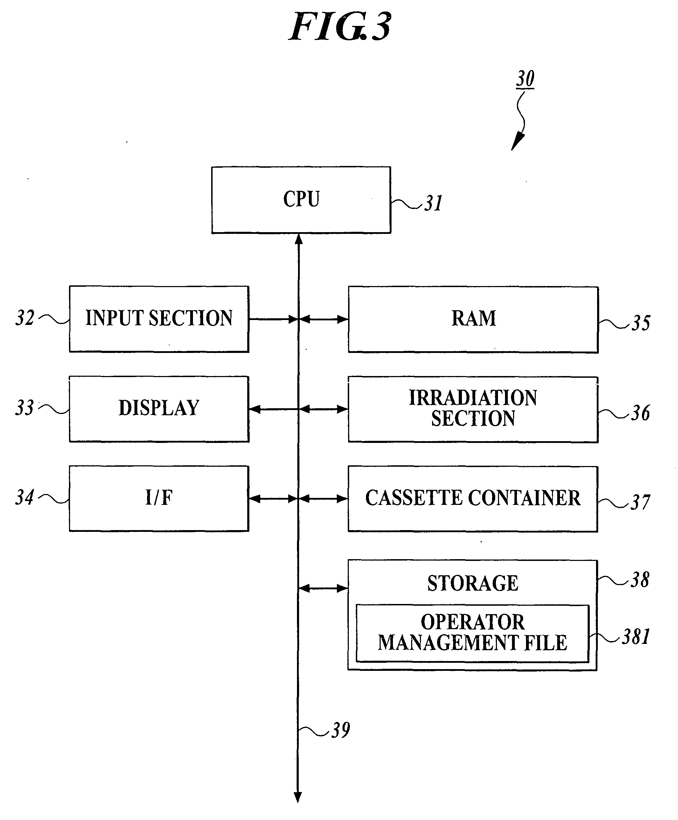

FIG. 3 is a block diagram showing the functional configuration of the radiographing apparatus 30. As shown in FIG. 3, the medical image radiographing apparatus 30 comprises a CPU 31, an input section 32, a display 33, a communication control section (I/F) 34, an RAM 35, an irradiation section 36, a cassette container 37 and a storage 38. Each of the constitutional sections of the radiographing apparatus 30 is connected to a bus 39.

The CPU 31 loads the system program and various control programs from the storage 38, expands the programs in the RAM 35 and controls intensively the operation of each element of the radiographing apparatus 30 according to the corresponding control program. Further, the CPU 31 performs various types of processing according to the programs expanded in the RAM 35, temporarily stores processing results in the RAM 35 and makes the display 33 display the processing results.

In detail, the CPU 31 loads the medical image radiographing processing program and performs the medical image radiographing processing (refer to FIG. 5) described later to act as an authentication section and a radiographing operation control section.

To perform the medical image radiographing processing, when the CPU 31 receives the operator ID from the PDA 10 connected to the cable B through the I/F 34, the CPU 31 obtains an operator management file 381 described later from the storage 38 and searches the operator management file 381 for the corresponding operator ID. When the CPU 31 can retrieve the corresponding operator ID from the operator management file 381, the CPU 31 judges that the operator is a regular operator, and the CPU 31 transmits an authentication result indicating the successful authentication to the PDA 10.

Further, when the CPU 31 receives information of the radiographing conditions from the PDA 10 through the I/F 34, the CPU 31 obtains the information of the radiographing conditions and sets the radiographing conditions. Further when the CPU 31 receives a radiographing instruction from the PDA 10 through the I/F 34, the CPU 31 makes the irradiation section 36 irradiate a subject (or a patient) with X-rays and radiograph the subject in response to the radiographing instruction. After the radiographing, the CPU 31 sets the correspondence of the radiographing order information and radiographing history information to the operator ID and stores them in the operator management file 381. The radiographing history information indicates the history of the radiographs taken by the operator and includes, for example, a date, resolution, sensitivity, a size, a direction, a radiation dose of X-rays and the like as conditions of the radiographing actually performed.

Further, when the CPU 31 receives an instruction indicating the finish of the radiographing from the PDA 10, the medical image radiographing processing is finished. Once the medical image radiographing processing is finished, unless the operator is successfully authenticated by again inputting the operator ID, the operator cannot radiograph the subject. In contrast, when the operator intends to successively radiograph the subject, the operator can successively radiograph the subject without authentication. However, when there is no input operation during a predetermined period of time (for example, around 15 minutes), the CPU 31 preferably makes the operator again input the operator ID to perform the authentication of the operator. Therefore, the security of the radiographing apparatus 30 can be further improved.

The input section 32 includes a keyboard having a cursor key, numeral input keys and various functional keys and outputs a push-down signal corresponding to a pushed-down key of the keyboard to the CPU 31. The input section 32 receives, for example, the radiographing conditions in the radiographing for the medical image, a radiographing instruction and the like. The input section 32 may comprise a pointing device such as a mouse, a touch panel or the like and another input apparatus, if necessary.

The display 33 comprises an LCD, a cathode ray tube (CRT) or the like and displays an inputted instruction and data transmitted from the input section 32 according to a display signal transmitted from the CPU 31.

The I/F 34 is an example of a reception section described according to the present invention and adjusts the form and the transfer rate of data transmitted and received through the cable B to intermediate the transmission and reception of the data between the radiographing apparatus 30 and the PDA 10. The connection of the I/F 34 with the PDA 10 is not limited to a wire, and the I/F 34 may be connected to the PDA 10 in wireless communication. In this case, the I/F 34 may have a data communication control function to control a data communication protocol according to the data communication method such as Infrared Data Association (IrDA) or the like to perform the short-range communication by using infrared rays. The communication method performing the wireless communication is not limited to IrDA, and the Bluetooth method may be, for example, adopted as the communication method performing the wireless communication.

The RAM 35 forms a storing area to temporarily store a system program and control programs read out from the storage 38 and capable to be executed in the CPU 31, input or output data, parameters and the like for each of various types of processing performed and controlled by the CPU 31.

The irradiation section 36 conically irradiates the subject with radiations under the control of the CPU 31 at both a radiation dose set according to the radiographing conditions and the irradiation timing instructed by the PDA 10 or the input section 32.

The cassette container 37 has a holding means to place the cassette 40 in the radiographing apparatus 30 to fix and protect the cassette 40. Further, because the medical image recorded in the photostimulable phosphor sheet due to the accumulated energy of the radiations is erased by irradiating the photostimulable phosphor sheet with light after the radiographing, the cassette container 37 prevents the erasure of the recorded medical image.

The storage 38 comprises a hard disc drive (HDD), a non-volatile semiconductor memory or the like and stores the programs (the system program and various processing programs corresponding to the system program) executed in the CPU 31 and processing results. The storage 38 further has a storage medium (not shown) to record programs and data in advance. The storage medium comprises a magnetic or optical storage medium or a semiconductor memory and is fixedly or detachably attached to the storage 38. The programs are stored in the storage 38 in the form of a readable program code, and the CPU 31 repeatedly performs an operation according to the program code.

The storage 38 is an example of a storage to store radiographing history information described according to the present invention and stores the operator management file 381 fo store the operator IDs, pieces of radiographing order information and pieces of radiographing history information while setting the correspondence of the radiographing order information and the radiographing history information to each operator ID. The operator management file 381 is used when the operator ID is checked for the authentication of the operator. Each operator ID registered in the operator management file 381 denotes an operator ID of an operator who is allowed in advance to operate the radiographing apparatus 30.

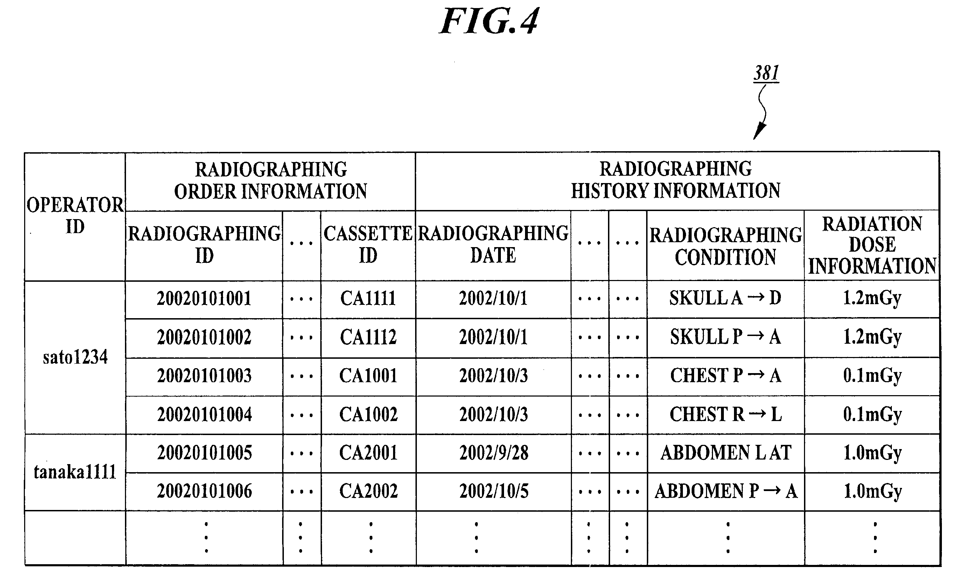

FIG. 4 is a view showing an example of the data construction of the operator management file 381. As shown in FIG. 4, the operator management file 381 stores the operator IDs, the pieces of radiographing order information and the pieces of radiographing history information. The operator of an operator ID "sato1234" performs the radiographing four times, and pieces of data of columns included in the radiographing order information and pieces of data of columns included in the radiographing history information are stored for each radiographing ID.

Next, an operation in this embodiment will be described hereinafter.

Each of the programs embodying functions described in flow charts described later are stored in the storage 17 of the PDA 10 or the storage 38 of the radiographing apparatus 30 in the form of a program code readable by a computer, and the CPU 11 of the PDA 10 or the CPU 31 of the radiographing apparatus 30 performs the operations one after another according to the program code.

FIG. 5 is a flow chart showing the medical image radiographing processing performed by the CPU 11 of the PDA 10. As shown in FIG. 5, when the operator ID is input through the operation section 12 or the barcode reader 18 (step S1), the CPU 11 transmits the inputted operator ID to the medical image radiographing apparatus 30 through the I/F 14 (step S2). The CPU 11 then receives an authentication result from the radiographing apparatus 30 through the I/F 14 (step S3) and judges whether or not the operator is successfully authenticated (step S4).

When the operator fails in authentication (NO in step S4), the procedure returns to the step S1, the CPU 11 urges the operator to again input his operator ID and performs the authentication of the operator according to the operator ID. When the operator fails in authentication a predetermined number of times (for example, three times), the medical image radiographing processing is preferably finished. In contrast, when the operator is successfully authenticated (YES in step S4), the CPU 11 urges the operator to input the radiographing conditions through the operation section 12 (step S5) and transmits the inputted radiographing conditions to the radiographing apparatus 30 through the I/F 14 (step S6). The CPU 11 preferably transmits the radiographing order information together with the radiographing conditions.

Thereafter, when a radiographing instruction is input through the operation section 12 (step S7), the CPU 11 transmits the radiographing instruction to the radiographing apparatus 30 through the I/F 14 to make the operator perform the radiographing according to the radiographing conditions while operating the radiographing apparatus 30 (step S8). The CPU 11 then urges the operator to input either an indication of the completion of the radiographing or an indication of the continuation of the radiographing. When the operator inputs the indication of the continuation of the radiographing (NO in step S9), the procedure proceeds to the step S5, and the CPU 11 urges the operator to input new radiographing conditions and makes the operator perform the radiographing according to the new radiographing conditions while operating the radiographing apparatus 30. In contrast, when the operator inputs the indication of the completion of the radiographing (YES in step S9), the CPU 11 ends the medical image radiographing processing.

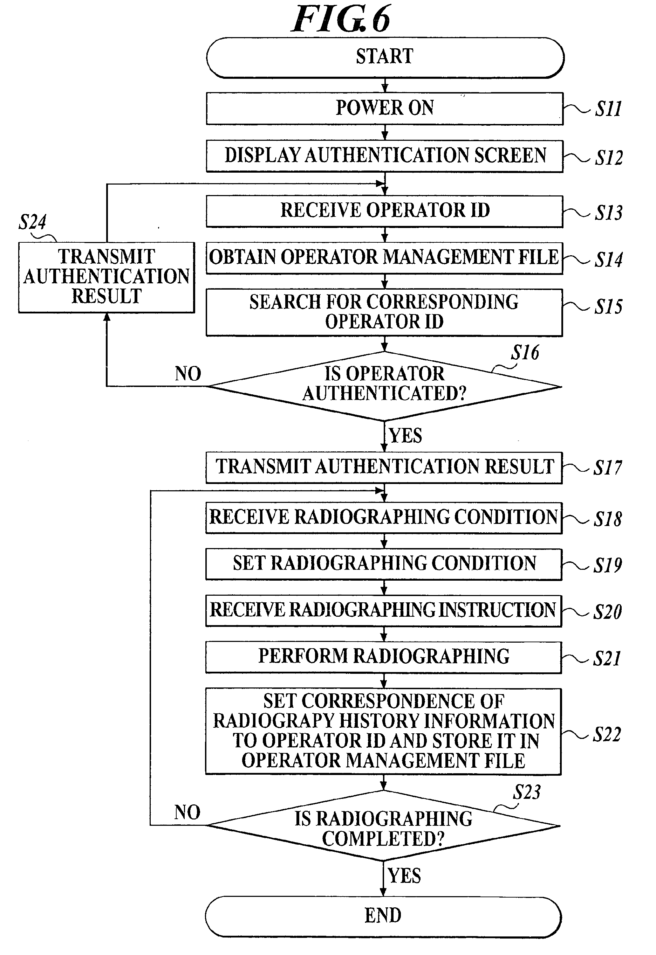

FIG. 6 is a flow chart showing the medical image radiographing processing performed by the CPU 31 of the radiographing apparatus 30. As shown in FIG. 6, when receiving an indication of the power supply, the CPU 31 loads a start program from the storage 38 and performs the start processing for the radiographing apparatus 30 (step S11). The CPU 31 then controls the display 33 to display an authentication screen (step S12) and urges the operator to input the operator ID through the PDA 10.

When receiving the operator ID through the I/F 34 (step S13), the CPU 31 obtains the operator management file 381 from the storage 38 (step S14) and searches the file 381 for the corresponding operator ID (step S15). When the corresponding operator ID is not registered in the operator management file 381, the CPU 31 judges that the operator fails. in authentication (NO in step S16) and transmits an authentication result indicating the failure of the authentication to the PDA 10 (step S24). The procedure proceeds to the step S13, and the CPU 31 urges the operator to again input the operator ID. The CPU 31 then performs the authentication of the operator according to the again-inputted operator ID.

In contrast, when the corresponding operator ID is registered in the operator management file 381, the CPU 31 judges that the operator is successfully authenticated (YES in step S16) and transmits an authentication result indicating the success of the authentication to the PDA 10 (step S17). Thereafter, the CPU 31 receives the information of the radiographing conditions from the PDA 10 (step S18) and performs the setting of the radiographing conditions according to the received information of the radiographing conditions (step S19). When receiving the radiographing instruction from the PDA 10 through the I/F 34 (step S20), the CPU 31 controls the irradiation section 36 according to the set radiographing conditions and makes the irradiation section 36 irradiate the subject with radiations to perform the radiographing according to the radiographing conditions (step S21).

Further, the CPU 31 controls the operator management file 381 to store the operator ID and the radiographing history information while setting the correspondence of the radiographing history information to the operator ID (step S22). In addition, the operator management file 381 may store the radiographing order information while setting the correspondence of the radiographing order information to the operator ID. The CPU 31 then judges whether or not the radiographing should be finished (step S23). When receiving the indication of the completion of the radiographing from the PDA 10, the CPU 31 judges that the radiographing should be finished (YES in step S23), and the CPU 31 ends the medical image radiographing processing. In contrast, when receiving the indication of the continuation of the radiographing from the PDA 10, the CPU 31 judges that the radiographing should be continued (NO in step S23), and the CPU 31 repeatedly performs the medical image radiographing processing.

As described above, the medical image radiographing system 1, which radiographs the patient at the visited patient's position while operating the movable radiographing apparatus 30 to obtain the medical image, urges the operator to input the operator ID specifying the operator to the PDA 10 connected to the radiographing apparatus 30 and makes the radiographing apparatus 30 perform the authentication of the operator according to the operator ID. When the radiographing apparatus 30 successfully authenticates the operator, the radiographing system 1 urges the operator to perform the radiographing while operating the radiographing apparatus 30 through the PDA 10. When the operator performs the radiographing while operating the radiographing apparatus 30, the radiographing apparatus 30 sets the correspondence of the radiographing history information including the date of the radiographing, the radiographing conditions and the like to the operator ID of the operator and stores the radiographing history information and the operator ID in the operator management file 381.

Therefore, even though the radiographing apparatus 30 is carried to a visited patient's position so as to set the radiographing apparatus 30 in a condition operable by a person other than the operator, unless the operator is correctly authenticated by inputting his/her operator ID registered in advance, the radiographing apparatus 30 cannot be operated. That is, when the operator fails in authentication or the authentication of the radiographer is not performed, even though the radiographing instruction is inputted through the operation section 12 of the PDA 10 or the input section 32 of the radiographing apparatus 30, the reception of the radiographing instruction is rejected in the radiographing apparatus 30, and there is no probability that the radiographing apparatus 30 is operated according to the radiographing instruction. Accordingly, an imprudent operation of the radiographing apparatus 30 by a third person can be prevented, and the radiographing can be safely performed even at the visited patient's position to obtain the medical image.

Further, when the operator performs the radiographing after the authentication of the operator, the radiographing history information and the operator ID are stored in the operator management file 381 while setting the correspondence of the radiographing history information to the operator ID. Accordingly, the history of the radiographs taken by the operator while operating the radiographing apparatus 30 can be stored. Thereby, when wrong medical treatment such as the radiographing of a wrong patient, the radiographing of a patient according to a radiographing instruction to radiograph another patient or the like is, for example, performed, the cause of the occurrence of the wrong medical treatment can be immediately investigated, and the reoccurrence of the wrong medical treatment can be prevented. Further, because the radiographing history information is recorded, the occurrence of the wrong medical treatment for the patients can be suppressed.

Moreover, because the operator ID is input by using the barcode reader 18, a mistake about the input operation can be prevented, the troublesomeness in the authentication can be mitigated, and the operator can efficiently perform the radiographing to obtain the medical image. Further, because the operator ID can be input through the operation section 12, the operator ID can be input by using an appropriate input section according to the situation. Accordingly, the radiographing system 1 and the radiographing apparatus 30 can be operated at excellent convenience.

More further, because the radiographing conditions and the radiographing instruction can be input by using the PDA 10, the operability of the radiographing apparatus 30 can be improved. Further, because the configuration of the input section of the radiographing apparatus 30 can be simplified to minimize the radiographing apparatus 30, the radiographing apparatus 30 can be easily carried, and the burden of the operator can be mitigated.

An example of the medical image terminal and the medical image radiographing system preferable in the present invention is described in this embodiment, though not exclusively.

In this embodiment, when the operator radiographs the patient at the visited patient's position, the portable PDA 10 applied to the medical image terminal and the radiographing apparatus 30 applied to a movable radiographing apparatus are, for example, described. However, the present invention is not limited to this example. For example, the medical image terminal may comprise a general personal computer (PC), and the radiographing apparatus may comprise a radiographing apparatus fixedly arranged in the radiographing room. Further, the PDA 10 may comprise a portable communication terminal such as a portable telephone, a note type personal computer or the like.

The operator ID having a plurality of numerals combined with one another and the barcode of the operator ID are applied as identification information of the operator. However, the present invention is not limited to this embodiment. For example, a method to identify the operator by data of his/her name or birth day or the like and identifying the operator by data of his/her fingerprint, the palm-print of his/her hand, his/her voiceprint, his/her face, his/her iris or his/her ID card or the like may be applied. To perform this method, the input section of the PDA 10 preferably receives the pieces of data. Further, the operator may be identified by the combination of pieces of information. Accordingly, the security of the medical image radiographing system can be improved by performing the authentication of the operator according to the operator ID with more accurate.

Further, the authentication of the operator may be performed according to identification information peculiar to the PDA 10 such as serial numbers or the like transmitted to the radiographing apparatus 30 as the identification information of the operator. When each operator has his own PDA 10, the operator can be specified by the serial numbers of the PDA 10, and the troublesomeness of inputting the operator ID can be mitigated. Further, the authentication of the operator may be performed according to both the operator ID and the serial numbers of the PDA 10. In this case, a stronger security system can be obtained.

The pieces of information stored in the operator management file 381 are described as an example, and the present invention is not limited to this example. For example, the radiographing order information may comprises a name of a doctor in charge, warning information indicating the warning of an infectious disease, the/no existence of a drug allergy, pregnancy or not, a clinical history, the/no necessity of special nursing such as the/no necessity of a wheel chair, the/no necessity of a stretcher or the like, a clinically diagnosed disease, privileged information to be protected, a radiographing method (simple radiography, contrast radiography or the like), an expected date and the like. Further, the configuration that the radiographing order information is not stored in the operator management file 381 may be applied.

Further, the radiographing apparatus 30 may make the information managing apparatus 20 perform the authentication of the operator. Moreover, the information managing apparatus 20 may perform the authentication of the operator by directly communicating with the PDA 10.

It will be appreciated by those skilled in the art that modifications of the configuration and function of each constitutional element of the medical image radiographing system 1 may be made to the embodiment hereinbefore described without departing from the scope of the present invention.

According to the present invention, when the medical image radiographing apparatus is operated, the medical image radiographing apparatus obtains the identification information of the radiographer through the medical image terminal. When the medical image radiographing apparatus succeeds in the authentication of the radiographer, the radiographing of the medical image is performed according to the radiographing instruction transmitted from the medical image terminal. Accordingly, the radiographing of the medical image according to a radiographing instruction other than the radiographing instruction registered as the radiographer in advance is prohibited, and the medical image radiographing apparatus can be prevented from being carelessly operated by a third person.

Further, when the radiographer is not authenticated, even though the radiographing instruction is inputted from the input section of the medical image radiographing apparatus or the medical image terminal, the radiographing operation cannot be performed. Accordingly, the medical image radiographing apparatus can be prevented from being erroneously operated by a third person, and the medical image radiographing apparatus can be safely handled.

Further, because the radiographing condition can be inputted to the medical image radiographing apparatus through the medical image terminal, the operability of the medical image radiographing apparatus is superior. Moreover, because the input section of the medical image radiographing apparatus can be miniaturized, when the radiographing is performed by carrying the medical image radiographing apparatus to the visited patients position, the medical image radiographing apparatus can be easily carried, and the radiographing of the medical image can be quickly performed.

Further, because the history of the radiographing performed by the radiographer is stored while setting the correspondence to the identification information of the radiographer, when wrong medical treatment is performed, the cause of the occurrence of the wrong medical treatment can be immediately examined, and the recurrence of the wrong medical treatment can be prevented. Moreover, because the history of the radiographing performed by the radiographer is stored, the effect of the suppression of the occurrence of the wrong medical treatment can be expected to the radiographer.

Further, because the medical image radiographing terminal is a portable terminal, each of the radiographers can bring his/her own medical image radiographing terminal. Accordingly, only the specific radiographer can operate his/her medical image radiographing terminal, and the security of the medical image radiographing system can be improved. Moreover, when the radiographing is performed at the visited patient's position, radiographing members can be easily carried by miniaturizing the medical image radiographing terminal, and the burden of the radiographer can be mitigated.

The entire disclosure of Japanese Patent Application No. Tokugan 2002-317228 filed on October 31, 2002 including specification, claims, drawings and summary are incorporated herein by reference in its entirety.

## Claims

1. A medical image radiographing system comprising:
a medical image radiographing apparatus to perform radiographing of a medical image by irradiating with radiations,
the medical image radiographing apparatus comprising:
an irradiation section to irradiate a subject with the radiations;
an obtaining section to obtain identification information of a radiographer and a radiographing instruction; and
a radiographing operation control section to control the irradiation section to perform a radiographing operation according to the radiographing instruction obtained by the obtaining section when the radiographer is authenticated according to the identification information of the radiographer obtained by the obtaining section.

2. The system of claim 1; wherein the obtaining section obtains the identification information of the radiographer and the radiographing instruction by inputting the identification information of the radiographer and the radiographing instruction.

3. The system of claim 1; further comprising a medical image terminal,
the medical image terminal comprising:
an input section to input the identification information of the radiographer; and
a transmission section to transmit the identification information of the radiographer inputted by the input section,
wherein the obtaining section obtains the identification information of the radiographer by receiving the identification information transmitted by the transmission section and obtains the radiographing instruction by inputting the radiographing instruction.

4. The system of claim 1; further comprising a medical image terminal, the medical image terminal comprising:
an input section to input the identification information of the radiographer and the radiographing instruction; and
a transmission section to transmit the identification information of the radiographer and the radiographing instruction inputted by the input section,
wherein the obtaining section obtains the identification information of the radiographer and the radiographing instruction by receiving the identification information and the radiographing instruction transmitted by the transmission section.

5. The system of claim 1, 2, 3 or 4; wherein the obtaining section of the medical image radiographing apparatus obtains a radiographing condition, and the radiographing operation control section controls the irradiation section to perform the radiographing operation according to the radiographing condition and the radiographing instruction obtained by the obtaining section.

6. The system of claim 3 or 4; wherein the input section of the medical image terminal inputs a radiographing condition,
the transmitting section of the medical image terminal transmits the radiographing condition,
the obtaining section of the medical image radiographing apparatus obtains the radiographing condition by receiving the radiographing condition from the transmitting section, and
the radiographing operation control section of the medical image radiographing apparatus controls the irradiation section to perform the radiographing operation according to the radiographing condition obtained by the obtaining section and the obtained radiographing instruction.

7. The system of claim 6; wherein the medical image radiographing apparatus further comprises a storage to store radiographing history information including the radiographing condition of the radiographing operation while setting the correspondence of the radiographing history information to the identification information of the radiographer when the radiographing operation is performed under control of the radiographing operation control section.

8. The system of any one of claims 1 to 7; wherein the medical image terminal is a portable terminal.

9. The system of any one of claims 1 to 8; further comprising:
an authentication section to perform authentication of the radiographer according to the identification information of the radiographer obtained by the obtaining section.

10. A medical image radiographing apparatus to perform the radiographing of a medical image by irradiating with radiations,
the medical image radiographing apparatus comprising:
an irradiation section to irradiate a subject with the radiations;
an obtaining section to obtain identification information of a radiographer and a radiographing instruction; and
a radiographing operation control section to control the irradiation section to perform a radiographing operation according to the radiographing instruction obtained by the obtaining section when the radiographer is authenticated according to the identification information of the radiographer obtained by the obtaining section.

11. The apparatus of claim 10; wherein the obtaining section obtains the identification information of the radiographer and the radiographing instruction by inputting the identification information and the radiographing instruction.

12. The apparatus of claim 10; wherein the obtaining section obtains the identification information of the radiographer by receiving the identification information and obtains the radiographing instruction by inputting the radiographing instruction.

13. The apparatus of claim 10; wherein the obtaining section obtains the identification information of the radiographer and the radiographing instruction by receiving the identification information and the radiographing instruction.

14. The apparatus of claim 10, 11, 12 or 13; wherein the obtaining section obtains a radiographing condition by inputting the radiographing condition, and the radiographing operation control section controls the irradiation section to perform the radiographing operation according to the radiographing condition and the radiographing instruction obtained by the obtaining section.

15. The apparatus of claim 10, 11, 12, 13 or 14, further comprising a storage to store radiographing history information including a radiographing condition of the radiographing operation while setting the correspondence of the radiographing history information to the identification information of the radiographer when the radiographing operation is performed under control of the radiographing operation control section.

16. A medical image radiographing method for radiographing a medical image by irradiating with radiations,
the medical image radiographing method comprising:
obtaining identification information of a radiographer;
performing authentication of the radiographer according to the obtained identification information of the radiographer;
obtaining a radiographing instruction; and
performing a radiographing operation according to the obtained radiographing instruction by irradiating with the radiations when the radiographer is authenticated.

17. The method of claim 16, further comprising:
obtaining a radiographing condition, and
the performing of the radiographing operation includes performing the radiographing operation according to the obtained radiographing instruction and the obtained radiographing condition by irradiating with the radiations when the radiographer is authenticated.

18. The method of claim 16, further comprising:
inputting a radiographing condition;
transmitting the radiographing condition;
receiving the radiographing condition; and
irradiating with the radiations to perform the radiographing operation according to the received radiographing condition and the obtained radiographing instruction.

19. The method of claim 17 or 18, further comprising:
storing radiographing history information including the radiographing condition of the radiographing operation while setting the correspondence of the radiographing history information to the identification information of the radiographer when the radiographing operation is performed.

20. A program to make a computer
obtain identification information of a radiographer and a radiographing instruction;
perform authentication of the radiographer according to the obtained identification information of the radiographer; and
instruct to perform a radiographing operation according to the obtained radiographing instruction by irradiating with radiations when the radiographer is authenticated.

21. The program of claim 20, further making the computer
obtain a radiographing condition; and
instruct to perform the radiographing operation according to the radiographing condition and the radiographing instruction by irradiating with the radiations.

22. The program of claim 21, further making the computer perform
the storing of radiographing history information including the radiographing condition of the radiographing operation while setting the correspondence of the radiographing history information to the identification information of the radiographer when the radiographing operation is performed.
